(19) **Europäisches Patentamt / European Patent Office / Office européen des brevets**

(11) **EP 4 047 356 A1**

(12) # EUROPEAN PATENT APPLICATION
published in accordance with Art. 153(4) EPC

(43) Date of publication:
**24.08.2022 Bulletin 2022/34**

(21) Application number: **20877479.4**

(22) Date of filing: **16.10.2020**

(51) International Patent Classification (IPC):
***G01N 24/08*** (2006.01)

(52) Cooperative Patent Classification (CPC):
**G01N 24/08**

(86) International application number:
**PCT/JP2020/039008**

(87) International publication number:
**WO 2021/075519 (22.04.2021 Gazette 2021/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.10.2019 JP 2019190477**

(71) Applicants:
- **RIKEN**
  **Wako-shi**
  **Saitama 351-0198 (JP)**
- **NATIONAL AGRICULTURE AND FOOD RESEARCH ORGANIZATION**
  **Tsukuba-shi, Ibaraki 305-8517 (JP)**

(72) Inventors:
- **TAJIMA, Yusuke**
  **Wako-shi, Saitama 351-0198 (JP)**
- **YOKOTA, Hideo**
  **Wako-shi, Saitama 351-0198 (JP)**
- **KIKU, Yoshio**
  **Sapporo-shi, Hokkaido 062-0045 (JP)**
- **HAYASHI, Tomohito**
  **Sapporo-shi, Hokkaido 062-0045 (JP)**
- **MIKAMI, Osamu**
  **Sapporo-shi, Hokkaido 062-0045 (JP)**
- **NAGASAWA, Yuya**
  **Sapporo-shi, Hokkaido 062-0045 (JP)**

(74) Representative: **Graf von Stosch Patentanwaltsgesellschaft mbH Prinzregentenstraße 22 80538 München (DE)**

(54) **DETECTION METHOD AND DETECTION SYSTEM FOR MASTITIS IN DOMESTIC ANIMAL**

(57) A method, in accordance with an embodiment of the present invention, for testing for mastitis in a domestic animal includes the step of measuring, by nuclear magnetic resonance (NMR), a spin-spin relaxation time of milk collected from a quarter of the domestic animal.

FIG. 8

## Description

Technical Field

The present invention relates to a method and a system for testing for mastitis in a domestic animal.

Background Art

Economic losses in Japan due to mastitis, which is one of infectious diseases suffered by dairy cows, amount to 80 billion yen per year. Since early detection of mastitis can reduce the cost of treatment and inhibit decreased milk production, research and development for early detection of mastitis is underway.

For example, Patent Literature 1 discloses a method for diagnosing mastitis by measuring a somatic cell count in milk. Patent Literature 2 discloses a method for diagnosing mastitis by measuring an electrical conductivity of milk. Patent Literature 3 discloses a method for diagnosing mastitis by decomposing somatic cells in milk to produce cytokine and the like and thus activate somatic cells, and then measuring a somatic cell count. Patent Literature 4 discloses a method for diagnosing mastitis by measuring an electrical conductivity of centrifuged milk.

Citation list

[Patent Literature]

> [Patent Literature 1]
> Published Japanese Translation of PCT International Application, Tokuhyo, No. 2002-503097
> [Patent Literature 2]
> Japanese Patent Application Publication, Tokukai, No. 2010-230363
> [Patent Literature 3]
> Japanese Patent Application Publication, Tokukai, No. 2010-256231
> [Patent Literature 4]
> Japanese Patent Application Publication, Tokukai, No. 2011-33599

Summary of Invention

Technical Problem

Methods for diagnosing mastitis by somatic cell count measurement have a problem that devices for measuring a somatic cell count are expensive. Measurement of a somatic cell count by microscopic observation has a problem that the operation is complicated. Methods for diagnosing mastitis by measuring an electrical conductivity of milk are not suitable for early diagnosis of mastitis because the methods observe a change caused by an inflammatory reaction after invasion by a pathogen. Further, since a composition ratio of electrolytes contained in milk varies among quarters of udder of the same individual or among individuals, reproducibility in the measurement of an electrical conductivity of milk is poor. Thus, there has not been developed a technique that can easily detect mastitis in a domestic animal, such as a dairy cow, at an early stage.

It is an object of an aspect of the present invention to provide a test that can easily detect mastitis in a domestic animal, such as a dairy cow, at an early stage.

Solution to Problem

In order to attain the object, the present invention includes the following aspects. - A testing method for testing for mastitis in a domestic animal, said method including the step of: measuring, by nuclear magnetic resonance (NMR), a spin-spin relaxation time of milk collected from a quarter of the domestic animal. - A testing system for testing for mastitis in a domestic animal, including: a milking machine including a milker unit and a milk claw; and an NMR measurement device, a flow cell for NMR measurement being provided at a predetermined position between the milker unit and a position short of the milk claw.

Advantageous Effects of Invention

An aspect of the present invention makes it possible to provide a test that can easily detect mastitis in a domestic animal, such as a dairy cow, at an early stage.

Brief Description of Drawings

> Fig. 1 is a graph illustrating a result of analysis of milk collected from each quarter in Example 1.
> Fig. 2 is a graph illustrating a result of analysis of milk collected from each quarter in Example 2.
> Fig. 3 is a graph illustrating a result of analysis of milk collected from each quarter in Example 3.
> Fig. 4 is a graph illustrating a result of analysis of milk collected from each quarter in Example 4.
> Fig. 5 is a graph illustrating a result of analysis of milk by addition of *Staphylococcus aureus* (SA-VB) in Evaluation Example 1.
> Fig. 6 is a graph illustrating a result of analysis of milk by addition of *Staphylococcus aureus* (SA-VR) in Evaluation Example 1.
> Fig. 7 is a graph illustrating a result of analysis of milk by addition of lactic acid bacteria in Evaluation Example 2.
> Fig. 8 is a schematic view illustrating an example of a testing system in accordance with an embodiment of the present invention.
> Fig. 9 is a block diagram illustrating a configuration of a main part of a testing system in accordance with an embodiment of the present invention.

Fig. 10 is a graph illustrating a result of analysis of milk collected from each quarter in Example 5.

Fig. 11 is a graph illustrating a result of analysis of milk collected from each dairy cow in Example 6.

Fig. 12 is a graph illustrating a result of analysis of milk collected from each dairy cow in Example 7.

Fig. 13 is a graph illustrating a result of analysis of milk by addition of *Staphylococcus aureus* in Evaluation Example 3.

Description of Embodiments

[1. Method for testing for mastitis in domestic animal]

A method (hereinafter, also abbreviated as a "testing method of the present invention"), in accordance with an embodiment of the present invention, for testing for mastitis in a domestic animal includes the step of measuring, by nuclear magnetic resonance (NMR), a spin-spin relaxation time of milk (hereinafter, also referred to as "sample milk") collected from a quarter of the domestic animal.

Examples of the domestic animal include cattle such as a dairy cow, a goat, a buffalo, a sheep, and a horse. For example, cattle such as a dairy cow have four independent quarters: a right front quarter; a right rear quarter; a left front quarter; and a left rear quarter.

The nuclear magnetic resonance (NMR) used in the testing method of the present invention only needs to be capable of measuring a spin-spin relaxation time ($T_2$) of sample milk, and examples of the NMR include pulsed NMR. From perspectives such as reduction of a size of a measuring device, it is preferable to use pulsed NMR.

The milk can be collected from a quarter of the domestic animal by means ordinarily used by a person skilled in the art, such as a milking machine. By attaching an NMR measurement device to the milking machine, the spin-spin relaxation time ($T_2$) of the milk can be measured easily and non-invasively. In NMR (in particular, pulsed NMR), one measuring time is as short as several tens of seconds. Further, by attaching an NMR measurement device to an automatic milking machine, the spin-spin relaxation time ($T_2$) can be measured continuously at regular intervals. Further, by attaching an NMR measurement device to an automated milking machine, there is no need to sample milk for testing for mastitis, and the burden on a manager of domestic animals can be reduced. The testing method of the present invention therefore allows real-time measurement (testing). Note that it is preferable to have the above configuration in which an NMR measurement device is attached to a milking machine and a portion of the milking machine is used as a flow cell for NMR measurement. However, the present invention is not particularly limited to such a configuration.

The spin-spin relaxation time or a specific surface area of particles contained in the milk, which specific surface area is obtained by converting the spin-spin relaxation time, may be compared with a predetermined standard S1. The comparison allows determining whether or not the quarter from which the milk was collected has mastitis or is likely to have mastitis. Specifically, measured data (the spin-spin relaxation time or the specific surface area) of the sample milk is compared with the predetermined standard S1, and in a case where the measured data of the sample milk is significantly increased or decreased in comparison to the predetermined standard S1, it is possible to determine whether or not the quarter from which the sample milk was collected has mastitis or is likely to have mastitis. When comparing the data of the sample milk with the predetermined standard S1, whether or not there is a significant difference can be determined by a statistical method such as, for example, a t-test, an F-test, a chi-square test, or a Mann-Whitney's U test.

The specific surface area of the particles contained in the milk can be obtained from the following equation (1).

$$(1): \mathrm{Rav} = \Psi \mathrm{pSL}\rho\mathrm{p}(\mathrm{Rs}\text{-}\mathrm{Rb})\text{+}\mathrm{Rb}$$

In equation (1), Rav is a reciprocal of an average relaxation time. Ψp is a volume fraction (volume concentration) of the particles contained in the milk. L is a thickness (wet thickness) of a liquid layer adsorbed on surfaces of the particles contained in the milk. ρp is a density of the particles contained in the milk. Rs is a reciprocal of a relaxation time of molecules of a liquid adsorbed on the surfaces of the particles contained in the milk. Rb is a reciprocal of a relaxation time of solvent water.

Examples of the predetermined standard S1 include: (1) data of milk collected, from the quarter from which the sample milk was collected, at a time point (e.g., in advance) different from a time point at which the sample milk was collected; (2) data of milk collected from another quarter of the individual from which the sample milk was collected; and (3) data of milk collected from a quarter of an individual different from the individual from which the sample milk was collected. The data (1) through (3) are each a spin-spin relaxation time of the milk or a specific surface area of particles contained in the milk, which specific surface area is obtained by converting the spin-spin relaxation time. The data (2) and (3) may each be data of milk collected simultaneously with the collection of the sample milk, or data of milk collected in advance of the collection of the sample milk. The predetermined standard S1 can be determined such that: a spin-spin relaxation time (specific surface area) of milk collected in advance from a control quarter having no mastitis is measured; and a normal range (e.g., a standard S1 that serves as a threshold) is determined on the basis of the spin-spin relaxation time (specific surface area).

The predetermined standard S1 may be, for example, a collection of time-series data obtained at a plurality of time points with respect to the quarter from which the sample milk was collected. In this case, an evaluation is made of a change over time in the spin-spin relaxation time of milk from the quarter from which the sample milk was collected, or a change over time in the specific surface area of the particles contained in the milk, which specific surface area is obtained by converting the spin-spin relaxation time.

The testing method of the present invention may further include a step of comparing a somatic cell count in the sample milk with a predetermined standard S2. When comparing the somatic cell count of the sample milk with the predetermined standard S2, whether or not there is a significant difference can be determined by a statistical method such as, for example, a t-test, an F-test, a chi-square test, or a Mann-Whitney's U test. In the present specification, "a somatic cell count contained in milk" refers to a total number of blood-derived cells (leukocytes and the like) and mammary gland epithelial cells contained in the milk. The somatic cell count can be measured by a well-known method.

Examples of the predetermined standard S2 include: (4) a somatic cell count in milk collected, from the quarter from which the sample milk was collected, at a timing (e.g., in advance) different from a timing at which the sample milk was collected; (5) a somatic cell count in milk collected from another quarter of the individual from which the sample milk was collected; and (6) a somatic cell count in milk collected from a quarter of an individual different from the individual from which the sample milk was collected. The above data (5) and (6) may each be a somatic cell count in milk collected simultaneously with the collection of the sample milk, or a somatic cell count in milk collected in advance of the collection of the sample milk. The predetermined standard S2 can be determined such that: a somatic cell count in milk collected in advance from a control quarter having no mastitis is measured; and a normal range (e.g., a standard S2 that serves as a threshold) is determined on the basis of the somatic cell count.

The predetermined standard S2 may be, for example, a collection of time-series data obtained at a plurality of time points with respect to the quarter from which the sample milk was collected. In this case, an evaluation is made of a change over time in the somatic cell count in milk from the quarter from which the sample milk was collected, or a change over time in the specific surface area of the particles contained in the milk, which specific surface area is obtained by converting the spin-spin relaxation time.

As indicated in Examples, in a case where mastitis has developed due to infection with Gram-positive bacteria such as *Staphylococcus aureus,* a specific surface area of particles contained in milk decreases as a somatic cell count increases. This is considered to be because an acidic substance (e.g., lactic acid) produced by the Gram-positive bacteria causes casein micelles, which are dispersed in milk, to aggregate. In contrast, in a case where mastitis (e.g., mastitis due to infection with Gram-negative bacteria such as *E. coli,* mastitis due to trauma, or the like), which is other than mastitis due to infection with Gram-positive bacteria, has developed, a specific surface area of particles contained in milk increases as a somatic cell count increases. As such, by carrying out somatic cell measurement in addition to NMR measurement, it is possible to presume a cause of mastitis (whether or not the mastitis is caused by Gram-positive bacterial infection).

[2. System for testing for mastitis in domestic animal]

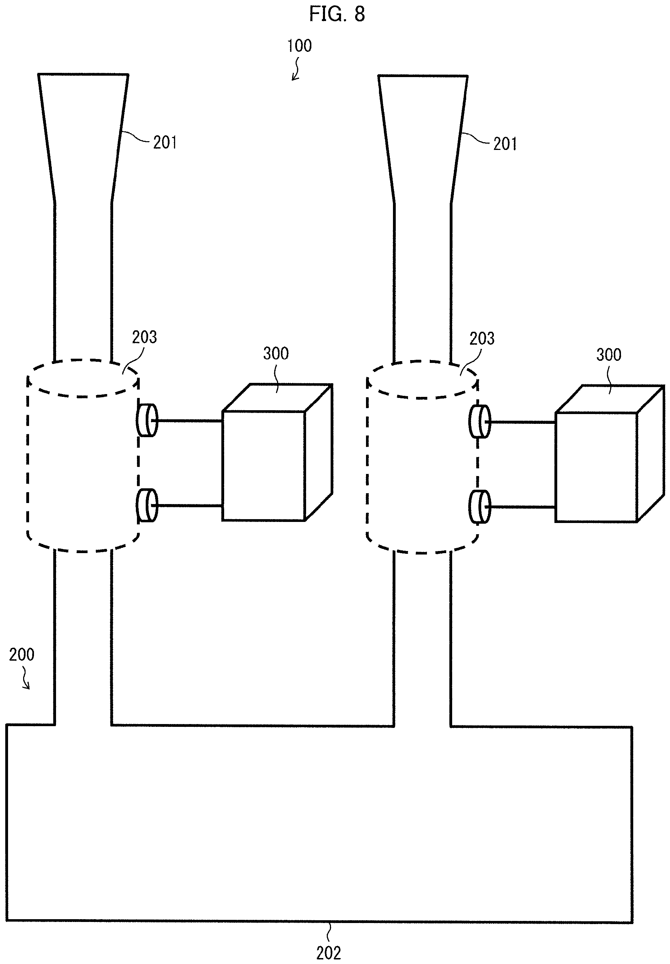

With reference to Fig. 8, the following description will discuss a system (hereinafter, also abbreviated as a "testing system of the present invention") 100, in accordance with an embodiment of the present invention, for testing for mastitis in a domestic animal. The testing system 100 includes a milking machine 200 and an NMR measurement device 300. The milking machine 200 includes: a milker unit 201 which is attached to a quarter and takes in collected milk; a milk claw (milk mixing tank) 202 which mixes milk collected from respective quarters; and a flow cell 203 for NMR measurement.

The milking machine 200 can be a well-known milking machine. For example, in a case where four quarters of a dairy cow are tested simultaneously, four milker units may be provided. The flow cell 203 is installed in a predetermined position between the milker unit 201 and a position short of the milk claw 202. This allows a spin-spin relaxation time of milk collected from each quarter to be measured.

It is only necessary that the NMR measurement device 300 can be attached to the flow cell 203 of the milking machine 200. The NMR measurement device 300 can be, for example, a well-known NMR measurement device. From perspectives such as easy attachment to the milking machine 200 and reduction of a size of a measuring device, it is preferable that the NMR measurement device 300 be a pulsed NMR measurement device.

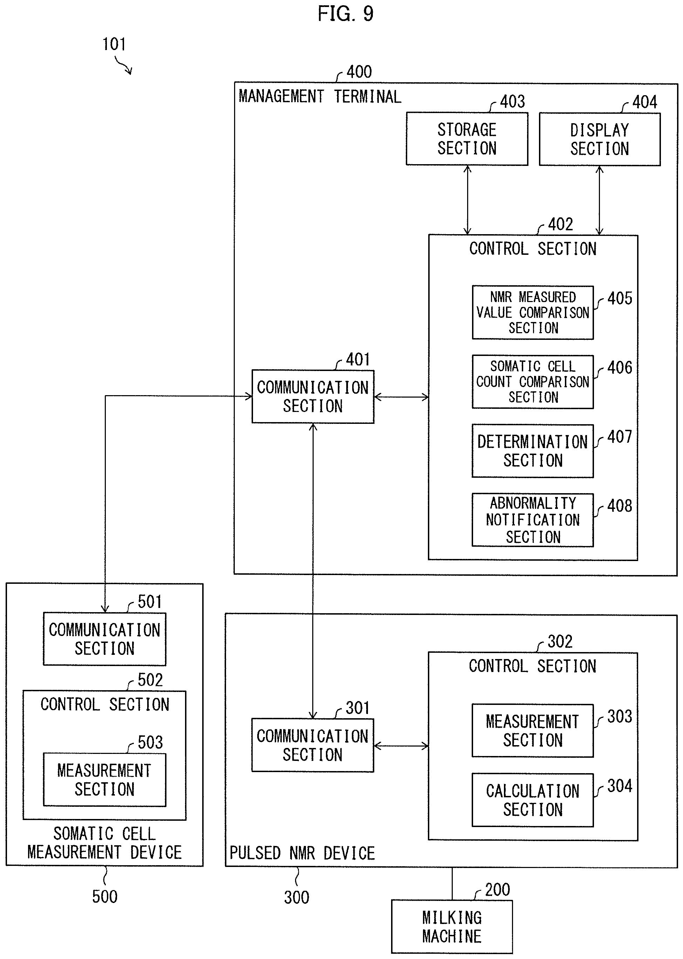

With reference to Fig. 9, the following description will discuss a testing system 101, which is another embodiment, of the present invention. Fig. 9 is a block diagram illustrating a configuration of a main part of a testing system. The testing system 101 includes a milking machine 200, an NMR measurement device 300, a management terminal (e.g., a small mobile terminal such as a smartphone) 400, and a somatic cell measurement device 500.

The NMR measurement device 300 includes a communication section 301, a measurement section 303, a calculation section 304, and a control section 302 which controls the measurement section 303 and the calculation section 304. The communication section 301 communicates with the management terminal 400. The measurement section 303 measures a spin-spin relaxation time of milk collected from each quarter. On the basis of the spin-spin relaxation time, the calculation section 304 converts a specific surface area of particles contained in the milk.

The management terminal 400 includes a communication section 401, an NMR measured value comparison section 405, a somatic cell count comparison section 406, a determination section 407, an abnormality notification section 408, a storage section 403, a display section 404, and a control section 402. The communication section 401 communicates with the NMR measurement device 300 and the somatic cell measurement device 500. The NMR measured value comparison section 405 compares the spin-spin relaxation time or the specific surface area of the sample milk with the predetermined standard S1. The somatic cell count comparison section 406 compares the somatic cell count in the sample milk with the predetermined standard S2. The determination section 407 determines, on the basis of a result of comparison by the NMR measured value comparison section 405, whether or not the quarter from which the sample milk was collected has mastitis or is likely to have mastitis. In a case where the determination section 407 has determined that the quarter has mastitis or is likely to have mastitis, the abnormality notification section 408 provides notification of such to a user of the management terminal 400. The display section 404 displays the results of measurement by the NMR measurement device 300 and the somatic cell measurement device 500, as well as the notification by the abnormality notification section 408.

The determination section 407 may presume a cause of the mastitis (whether or not the mastitis is due to Gram-positive bacterial infection) on the basis of the result of the somatic cell count comparison section 406 in addition to the result of comparison by the NMR measured value comparison section 405.

The somatic cell measurement device 500 includes: a communication section 501 which communicates with the management terminal 400; a measurement section 503 which measures a somatic cell count in milk collected from each quarter; and a control section 502 which controls the measurement section 503.

The present invention is not limited to the embodiments, but can be altered by a skilled person in the art within the scope of the claims. The present invention also encompasses, in its technical scope, any embodiment derived by combining technical means disclosed in differing embodiments.

The present invention is not limited to the embodiments, but can be altered by a skilled person in the art within the scope of the claims. The present invention also encompasses, in its technical scope, any embodiment derived by combining technical means disclosed in differing embodiments.

[3. Recap of embodiments]

The embodiments of the present invention include, for example, the following aspects.

<1> A testing method for testing for mastitis in a domestic animal, said method including the step of: measuring, by nuclear magnetic resonance, NMR, a spin-spin relaxation time of milk (sample milk) collected from a quarter of the domestic animal.

<2> The testing method as set forth in <1>, further including comparing, with a predetermined standard S1, the spin-spin relaxation time or a specific surface area of particles contained in the milk, the specific surface area being obtained by conversion of the spin-spin relaxation time.

<3> The testing method as set forth in <1> or <2>, further including: the step of comparing a somatic cell count in the milk with a predetermined standard S2.

<4> The testing method as set forth in any one of <1> through <3>, wherein the domestic animal is a dairy cow.

<5> A testing system for testing for mastitis in a domestic animal, including: a milking machine including a milker unit and a milk claw; and an NMR measurement device, a flow cell for NMR measurement being provided at a predetermined position between the milker unit and a position short of the milk claw.

<6> The testing system as set forth in <5>, further including a somatic cell measurement device.

<7> The testing system as set forth in <5> or <6>, wherein the domestic animal is a dairy cow.

<8> The testing method as set forth in <2>, wherein whether or not mastitis has developed is presumed by comparison with the predetermined standard S1.

<9> The testing method as set forth in <3>, wherein a cause of mastitis is presumed by comparison with the predetermined standard S2.

<10> Note that the testing methods of <1> through <4> can be interpreted as a method for obtaining data for carrying out testing for mastitis in a domestic animal and a method for analyzing the data.

Examples

A dairy cow has four quarters. Each quarter is an independent organ. Accordingly, milk produced by respective quarters differs in concentration of a component and the like, depending on a health condition of each quarter. Further, mastitis develops individually in each quarter. As such, in the following Examples, analysis was conducted with respect to milk collected from each quarter of a dairy cow. In Examples 1 and 2, in a case where a somatic cell count in milk was not less than $20 \times 10^4$/mL, it was determined that a quarter from which the milk was collected had mastitis. In Examples 3 and 4, in a case where a somatic cell count in milk was not less than

$50 \times 10^4$/mL, it was determined that a quarter from which the milk was collected had mastitis.

[Example 1] Analysis of milk collected from each quarter

Milk was collected from each of the four udder quarters (right front, right rear, left front, and left rear beasts) of a research dairy cow A kept and managed at the National Institute of Animal Health of the National Agriculture and Food Research Organization. A somatic cell count in the collected milk and a spin-spin relaxation time ($T_2$) of the collected milk were measured. The somatic cell count was measured by a cell counter DCC (manufactured by DeLaval). The collected milk was inverted and mixed, and then sucked into a dedicated cassette and inserted into a main body for measurement. The relaxation time ($T_2$) was measured at 25°C with a pulsed NMR device (manufactured by Xigo Nanotools, device name: ACORN AREA) with use of the milk which was put into an NMR tube as it was without diluting the milk or adding anything to the milk. A mode in which measurement of $T_2$ was conducted was a Carr-Purcell Meiboom-Gill sequence (CPMG) method.

On the basis of the relaxation time ($T_2$) thus measured, a specific surface area (S) of particles contained in the milk was calculated using the following equation (1):

$$(1): \ \mathrm{Rav} \ = \ \Psi \mathrm{pSL} \rho \mathrm{p(Rs-Rb)+Rb}$$

In equation (1), Rav is a reciprocal of an average relaxation time. Ψp is a volume fraction (volume concentration) of the particles contained in the milk. L is a thickness (wet thickness) of a liquid layer adsorbed on surfaces of the particles contained in the milk. ρp is a density of the particles contained in the milk. Rs is a reciprocal of a relaxation time of molecules of a liquid adsorbed on the surfaces of the particles contained in the milk. Rb is a reciprocal of a relaxation time of a solvent water.

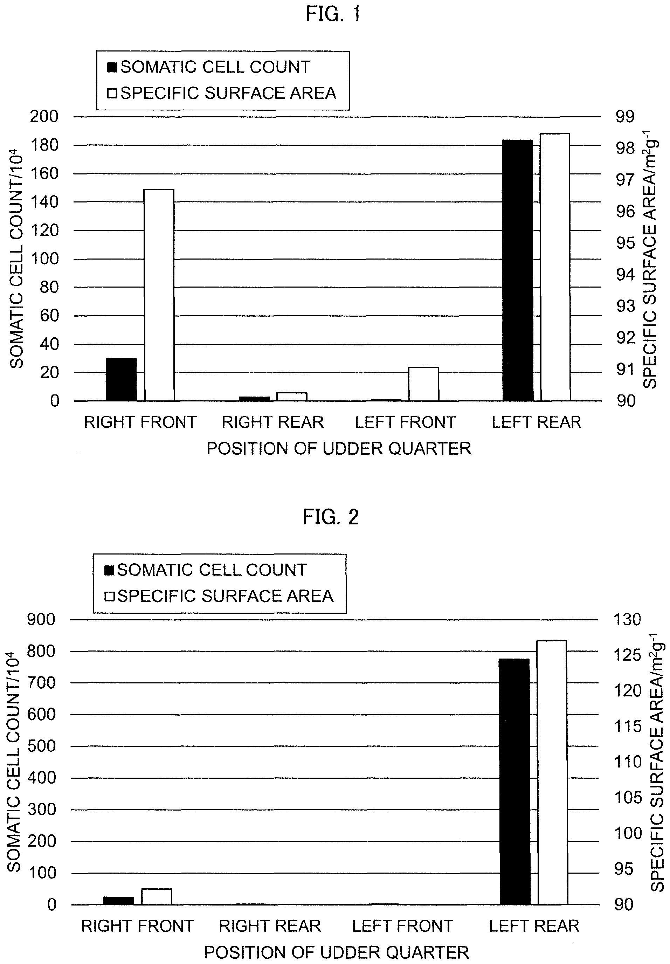

Fig. 1 illustrates results of the measured somatic cell count and the specific surface area calculated on the basis of the measured relaxation time ($T_2$). In Fig. 1, an axis on the left represents a somatic cell count ($10^4$) per milliliter. An axis on the right represents a specific surface area ($m^2$/g).

As illustrated in Fig. 1, somatic cell counts in milk respectively collected from the right rear quarter and the left front quarter were each not more than $20 \times 10^4$/mL, and the right rear quarter and the left front quarter were thus found to be healthy. In contrast, somatic cell counts in milk collected from the right front quarter and the left rear quarter were $30 \times 10^4$/mL and $182 \times 10^4$/mL, respectively, and it was thus indicated that both the right front quarter and the left rear quarter had mastitis. Neither milk was found by a bacteria test to have *Staphylococcus aureus* therein, and it was thus determined that the mastitis was due to *E. coli* or a trauma.

Further, a value of a specific area of particles was higher in the milk from the right front quarter and in the milk from the left rear quarter, as compared with the milk from the right rear quarter and the milk from the left front quarter. It was thus confirmed that a specific surface area corresponds to a somatic cell count.

[Example 2] Analysis of milk collected from each quarter

After the collection of milk in Example 1, the research dairy cow A was kept for 2 days, and then milk was collected again from each quarter. Then, a somatic cell count and a relaxation time ($T_2$) were measured and a specific surface area was calculated by the same procedure as in Example 1. Fig. 2 illustrates results of the measured somatic cell count and the specific surface area calculated on the basis of the measured relaxation time ($T_2$).

As illustrated in Fig. 2, somatic cell counts in milk respectively collected from the right rear quarter and the left front quarter were each not more than $20 \times 10^4$/mL, and the right rear quarter and the left front quarter were thus found to be healthy similarly as in the results of Example 1. A somatic cell count for the right front quarter was found to be on a healthier level in comparison to Example 1. The milk collected from the left rear quarter had a somatic cell count of $780 \times 10^4$/mL, and it was thus indicated that the left rear quarter had not recovered from mastitis. A value of a specific surface area of particles contained in milk was low in each of the milk from the right front quarter, the milk from the right rear quarter, and the milk from the left front quarter, whereas the value was high in the milk from the left rear quarter, corresponding to the somatic cell count.

[Example 3] Analysis of milk collected from each quarter

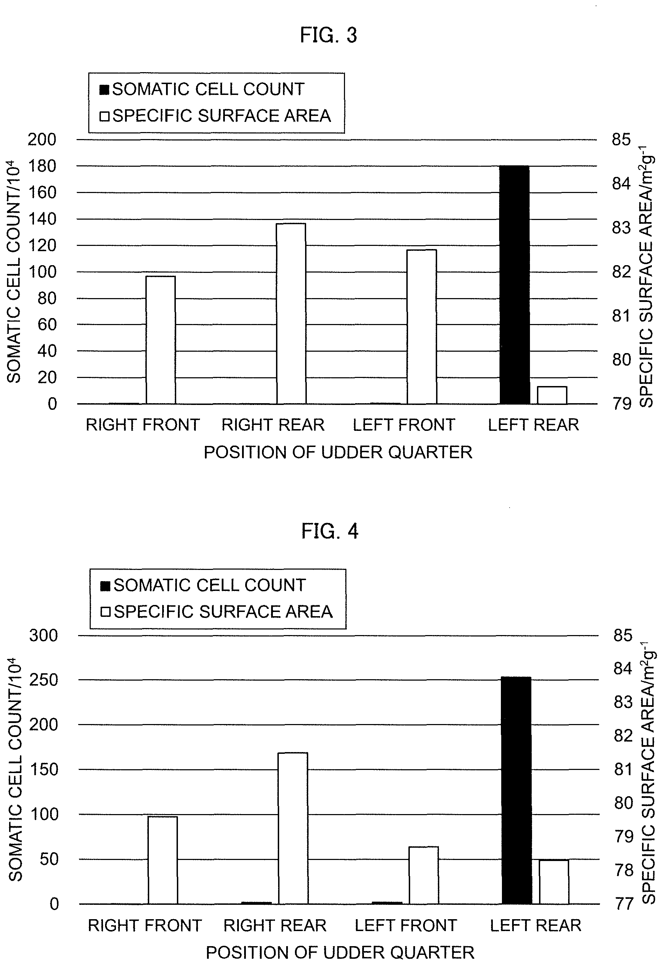

A somatic cell count and a relaxation time ($T_2$) were measured and a specific surface area was calculated by the same procedure as in Example 1, except that the dairy cow was changed to a research dairy cow B kept and managed at the National Institute of Animal Health of the National Agriculture and Food Research Organization. Fig. 3 illustrates results of the measured somatic cell count and the specific surface area calculated on the basis of the measured relaxation time ($T_2$).

As illustrated in Fig. 3, somatic cell counts in milk respectively collected from a right front quarter, a right rear quarter, and a left front quarter were each not more than $50 \times 10^4$/mL, and the right front quarter, the right rear quarter, and the left front quarter were thus found to be healthy. In contrast, a somatic cell count in milk collected from the left rear quarter was $180 \times 10^4$/mL, and it was thus indicated that the left rear quarter had mastitis. A bacteria test of the milk from the left rear quarter found *Staphylococcus aureus,* and it was thus determined that the mastitis developed in the left rear quarter was due to *Staphylococcus aureus.* A specific surface area of particles contained in the milk from the left rear quarter having mastitis was significantly lower than those in the milk from the right front quarter, the milk from the right rear quarter, and the milk from the left front quarter. This is because tycolic acid produced by Gram-positive bacteria such as *Staphylococcus aureus* caused micelles (such as casein) dispersed in milk to aggregate, so that the specific surface area was reduced.

[Example 4] Analysis of milk collected from each quarter

A somatic cell count and a relaxation time ($T_2$) were measured and a specific surface area was calculated by the same procedure as in Example 1, except that the dairy cow was changed to a research dairy cow C kept and managed at the National Institute of Animal Health of the National Agriculture and Food Research Organization. Fig. 4 illustrates results of the measured somatic cell count and the specific surface area calculated on the basis of the measured relaxation time ($T_2$).

As illustrated in Fig. 4, somatic cell counts in milk respectively collected from a right front quarter, a right rear quarter, and a left front quarter were each not more than $50 \times 10^4$/mL, and the right front quarter, the right rear quarter, and the left front quarter were thus found to be healthy. In contrast, a somatic cell count in milk collected from the left rear quarter was $250 \times 10^4$/mL, and it was thus indicated that the left rear quarter had mastitis. A bacteria test of milk from the left rear quarter found *Staphylococcus aureus,* and it was thus determined that the mastitis developed in the left rear quarter was due to *Staphylococcus aureus.* A specific surface area for the milk from the left rear quarter having mastitis was significantly lower than those for the right front quarter, the right rear quarter, and the left front quarter, and obtained results were thus similar to those in Example 3.

[Evaluation Example 1] Change in specific surface area of particles in milk caused by addition of *Staphylococcus aureus*

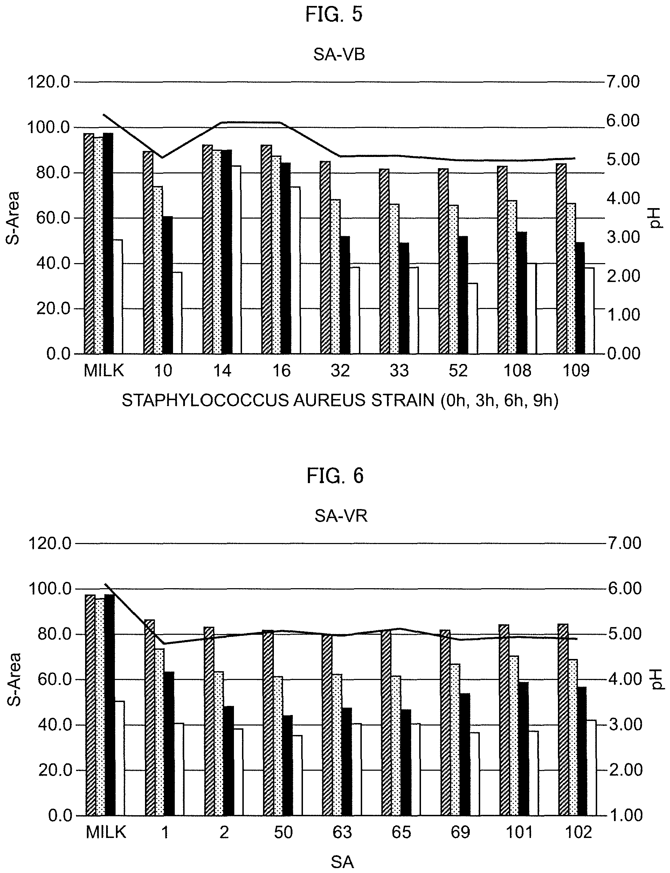

To 3 mL of milk collected from a healthy quarter, two different strains of *Staphylococcus aureus* (SA-VB and SA-VR) were each added in an amount of approximately 30 mg. The *Staphylococcus aureus* strains used were those preserved at the National Institute of Animal Health of the National Agriculture and Food Research Organization. SA-VR is more toxic than SA-VB. After *Staphylococcus aureus* was added, the milk was kept at 37°C. At 0, 3, 6, and 9 hours after the addition of *Staphylococcus aureus,* a relaxation time ($T_2$) of milk was measured and a specific surface area was calculated by the same procedure as in Example 1. A pH of the milk was also measured. Results of the measured pH and the specific surface area calculated on the basis of the measured relaxation time ($T_2$) are illustrated in Fig. 5 (SA-VB) and Fig. 6 (SA-VR).

In Figs. 5 and 6, an axis on the left represents a specific surface area ($m^2$/g). An axis on the right represents a pH. A horizontal axis represents a number of a *Staphylococcus aureus* strain used in Evaluation Example 1. "Milk" represents a control to which *Staphylococcus aureus* was not added. Four bars for each strain represent, from left, results of measurement of milk at 0, 3, 6, and 9 hours, respectively, after the addition of *Staphylococcus aureus.*

As illustrated in Figs. 5 and 6, a decrease in specific surface area was observed in milk with any type of *Staphylococcus aureus* added therein. It was also found that a degree of decrease in specific surface area differed depending on the pH of milk.

[Evaluation Example 2] Change in specific surface area of particles in milk caused by lactic acid fermentation

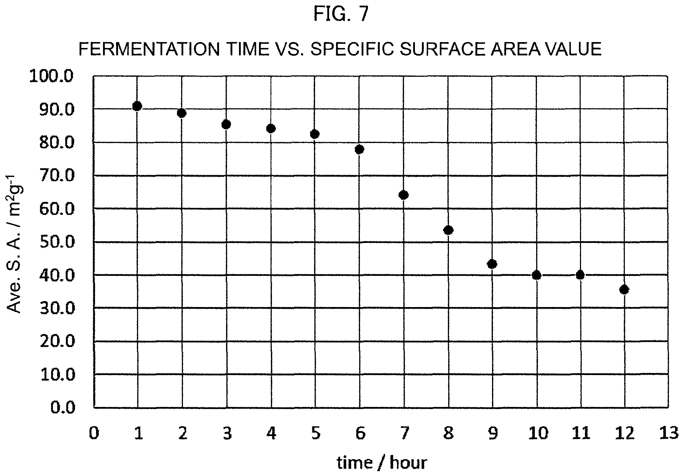

To 100 mL of milk collected from a healthy quarter, 1 g of fermented milk containing lactic acid bacteria was added. After the lactic acid bacteria was added, the milk was kept at 37°C. Every hour after the addition of the lactic acid bacteria, a relaxation time ($T_2$) of the milk was measured and a specific surface area was calculated by the same procedure as in Example 1. Fig. 7 illustrates results of calculation of the specific surface area on the basis of the measured relaxation time ($T_2$).

In Fig. 7, an axis on the left represents a specific surface area ($m^2$/g). A horizontal axis represents an elapsed time after the addition of the lactic acid bacteria. As illustrated in Fig. 7, a decrease in specific surface area was observed after the addition of the lactic acid bacteria, similarly as in the results of the addition of *Staphylococcus aureus* in Evaluation Example 1. This is because lactic acid produced by lactic acid fermentation caused casein micelles, which had been dispersed in milk, to lose a zeta potential and aggregate. It is considered that the decreases in specific surface area through the addition of *Staphylococcus aureus* in Evaluation Example 1 was also due to aggregation of casein micelles by lactic acid fermentation.

[Example 5] Analysis of milk collected from each quarter

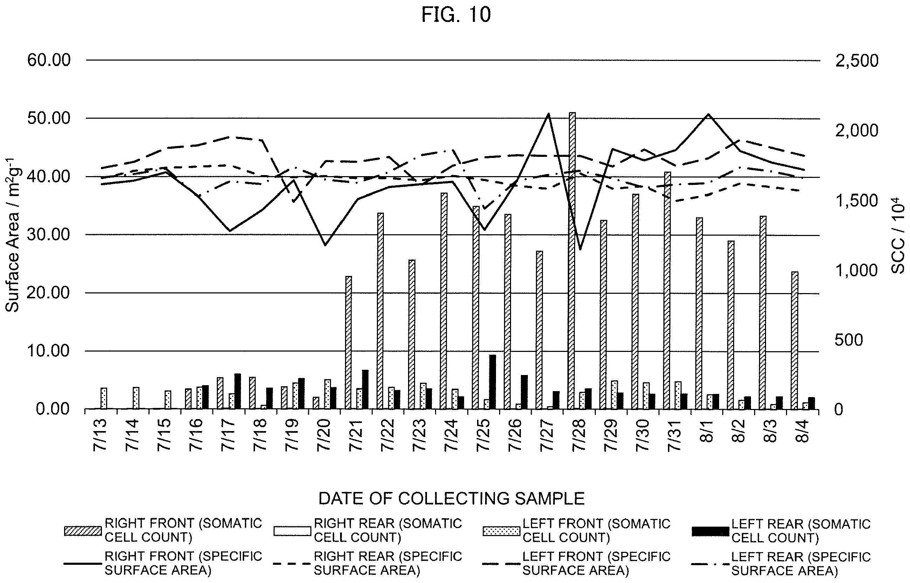

A somatic cell count and a relaxation time ($T_2$) were measured and a specific surface area was calculated by the same procedure as in Example 1, except that the dairy cow was changed to a research dairy cow D kept and managed at the National Institute of Animal Health of the National Agriculture and Food Research Organization. Fig. 10 illustrates results of the measured somatic cell count and the specific surface area calculated on the basis of the measured relaxation time ($T_2$).

In Fig. 10, an axis on the left represents a specific surface area ($m^2$/g), and an axis on the right represents a somatic cell count ($10^4$/mL). A line graph shows results of measurement of a specific surface area, and a bar graph shows results of measurement of a somatic cell count.

As illustrated in Fig. 10, a result of measurement on July 21 indicated that a somatic cell count for a right front quarter (RF) was approximately $1,000 \times 10^4$/mL, which was considerably higher than somatic cell counts for the other quarters. From this result, it was found that the right front quarter developed mastitis on July 21. Further, several days before July 21, a decrease in specific surface area of particles contained in milk from the right front quarter had been observed. It was thus determined that the mastitis that developed in the right front quarter was due to infection with *Staphylococcus aureus.* Further, several days before July 28, on which date the somatic cell count in the right front quarter considerably increased, a decrease had been observed in specific surface area of particles contained in milk from the right front quarter. The decrease in specific surface area of the particles contained in the milk is considered to be due to a decrease in pH and casein micelle aggregation in the milk which were caused by multiplication of *Staphylococcus aureus.*

[Example 6] Analysis of milk collected from each dairy cow

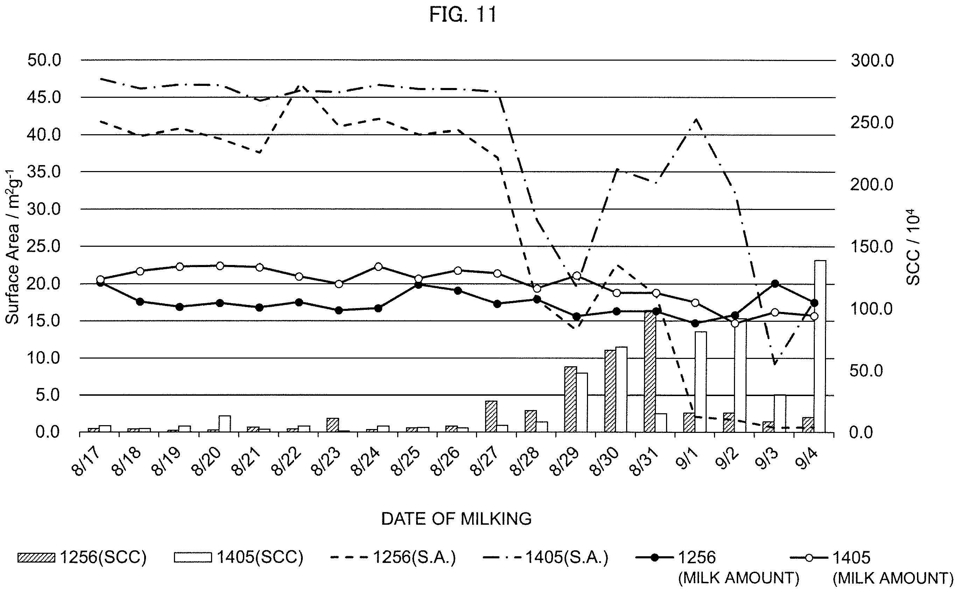

A somatic cell count and a relaxation time ($T_2$) were continuously measured and a specific surface area was calculated by the same procedure as in Example 1, with respect to a milk sample collected, directly with use of a catheter, from inside a quarter of each of two dairy cows kept at Rakuno Gakuen University. Fig. 11 illustrates results of the measured somatic cell count and the specific surface area calculated on the basis of the measured relaxation time ($T_2$).

In Fig. 11, an axis on the left represents a specific surface area ($m^2$/g), and an axis on the right represents a somatic cell count ($10^4$/mL). Two line graphs show results of measurement of a specific surface area (broken lines) and results of measurement of an amount of milk (solid lines), respectively, and a bar graph shows results of measurement of a somatic cell count.

As illustrated in Fig. 11, a somatic cell count in milk collected from a quarter of a dairy cow No. 1256 increased to not less than $100 \times 10^4$/mL on August 31. Before the increase in somatic cell count, a change in specific surface area of particles was observed in milk from the dairy cow No. 1256. Further, a somatic cell count in milk collected from a quarter of a dairy cow No. 1405 increased to not less than $100 \times 10^4$/mL on September 4. Also for the dairy cow No. 1405, a change in specific surface area of particles contained in milk was observed before the increase in cell count.

[Example 7] Analysis of milk collected from each dairy cow

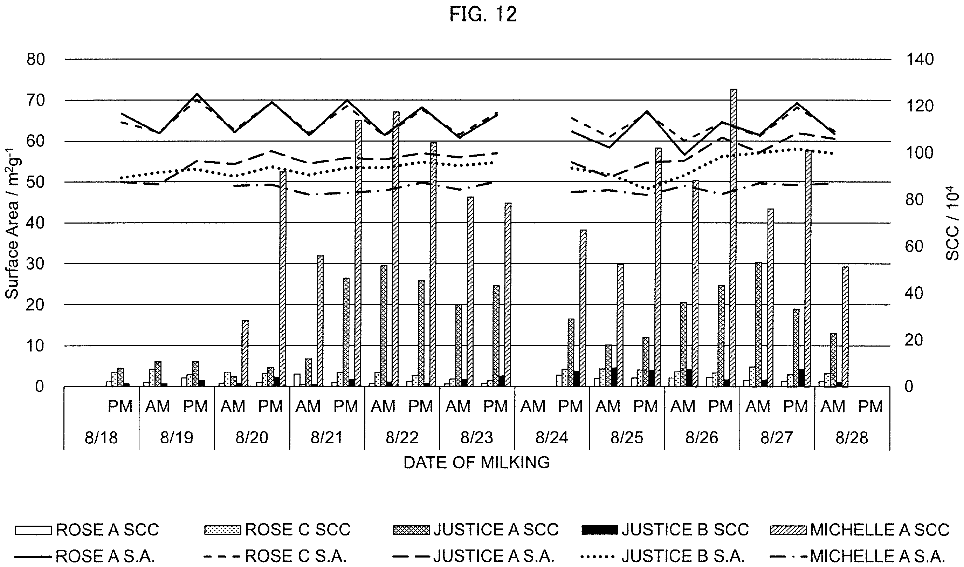

A somatic cell count and a relaxation time ($T_2$) were continuously measured and a specific surface area was calculated by same procedure as in Example 1, with respect to a milk sample collected from each of three dairy cows kept at Hokkaido Agricultural Technical College. Fig. 12 illustrates results of the measured somatic cell count and the specific surface area calculated on the basis of the measured relaxation time ($T_2$). Milk was collected twice a day, in the morning (AM) and evening (PM).

In Fig. 12, an axis on the left represents a specific surface area ($m^2$/g), and an axis on the right represents a somatic cell count ($10^4$/mL). Among the three dairy cows, Rose and Justice had their milk collected from two quarters each.

As illustrated in Fig. 12, with respect to all of the three dairy cows, it was found that a specific surface area of particles contained in milk changed periodically in the morning and evening. It is generally known that a calcium content is higher in milk collected in the evening than in milk collected in the morning. As such, it is considered that the specific surface area changed periodically because a casein micelle itself, which is a calcium medium, was contained in a greater amount in milk collected in the evening.

Similarly as in Fig. 11, there were some milk samples having a somatic cell count of more than $100 \times 10^4$/mL. However, no particular change in specific surface area was observed among the dairy cows. Particles contained in a milk sample (Justice and Michelle) in which the somatic cell count increased tended to have a lower specific surface area than particles contained in a milk samples in which the somatic cell count did not increase.

[Evaluation Example 3] Culture of *Staphylococcus aureus* in skim milk dispersion

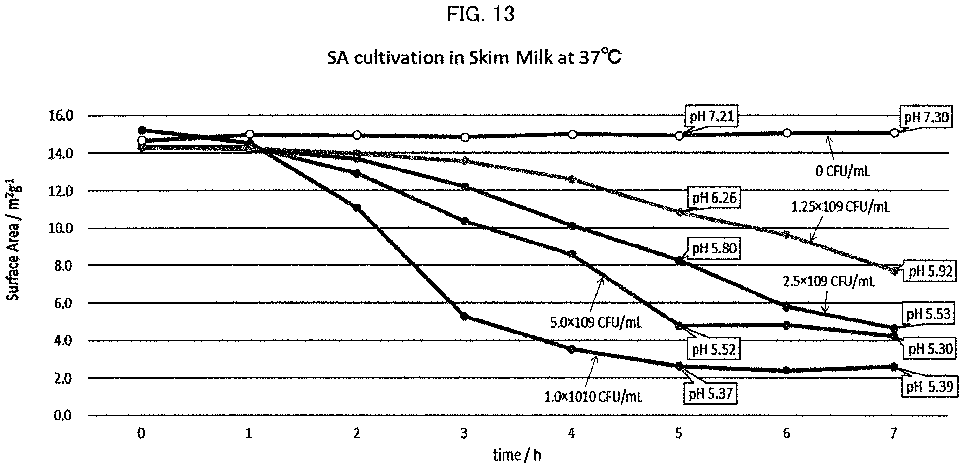

For quantitative in *vitro* observation of casein micelle aggregation caused by multiplication of *Staphylococcus aureus, Staphylococcus aureus* was added to 3% aqueous solutions of skim milk. In respective samples, a bacterial count was adjusted to $1.0 \times 10^{10}$ CFU/mL, $5.0 \times 10^9$ CFU/mL, $2.5 \times 10^9$ CFU/mL, and $1.25 \times 10^9$ CFU/mL, respectively. Then, the samples were each cultured at 37°C. Every hour after a start of the culturing, a relaxation time ($T_2$) of milk was measured and a specific surface area was calculated by the same procedure as in Example 1. Results of measurement are shown in Fig. 13.

In Fig. 13, a vertical axis represents a specific surface area ($m^2$/g), and a horizontal axis represents a culture time. As illustrated in Fig. 13, the greater an amount of *Staphylococcus aureus* added to a sample was, the earlier a decrease in specific surface area was observed in the sample. It was also observed that a pH of milk decreased in accordance with a decrease in specific surface area.

[Recap of Examples]

From the above results, it was found that, by using NMR to measure a spin-spin relaxation time (T$_2$) of milk collected from each quarter of a domestic animal such as a dairy cow, it is possible to determine whether or not the quarter had mastitis. It was also found that, by also carrying out somatic cell measurement simultaneously, it is possible to presume a type of mastitis (whether or not the mastitis is due to infection with Gram-positive bacteria such as *Staphylococcus aureus*).

Industrial Applicability

The present invention can be used in testing for mastitis in a domestic animal. Thus, the present invention can be widely used in fields such as food and medical diagnosis.

Reference Signs List

100, 101: testing system
200: milking machine
201: milker unit
202: milk craw
203: flow cell
300: NMR device
301, 401, 501: communication section
302, 402, 502: control section
303, 503: measurement section
304: calculation section
400: management terminal
403: storage section
404: display section
405: NMR measured value comparison section
406: somatic cell count comparison section
407: determination section
408: abnormality notification section
500: somatic cell measurement device

**Claims**

1. A testing method for testing for mastitis in a domestic animal,
said method comprising the step of:
measuring, by nuclear magnetic resonance, NMR, a spin-spin relaxation time of milk collected from a quarter of the domestic animal.

2. The testing method as set forth in claim 1, further comprising
comparing, with a predetermined standard S1, the spin-spin relaxation time or a specific surface area of particles contained in the milk, the specific surface area being obtained by conversion of the spin-spin relaxation time.

3. The testing method as set forth in claim 1 or 2, further comprising:
the step of comparing a somatic cell count in the milk with a predetermined standard S2.

4. The testing method as set forth in any one of claims 1 through 3, wherein the domestic animal is a dairy cow.

5. A testing system for testing for mastitis in a domestic animal, comprising:

a milking machine including a milker unit and a milk claw; and
an NMR measurement device,
a flow cell for NMR carried out by the NMR measurement device being provided at a predetermined position between the milker unit and a position short of the milk claw.

6. The testing system as set forth in claim 5, further comprising a somatic cell measurement device.

7. The testing system as set forth in claim 5 or 6, wherein the domestic animal is a dairy cow.

## FIG. 1

Legend: ■ SOMATIC CELL COUNT □ SPECIFIC SURFACE AREA

Left axis: SOMATIC CELL COUNT/$10^4$ (0–200)
Right axis: SPECIFIC SURFACE AREA/$m^2g^{-1}$ (90–99)
X-axis: POSITION OF UDDER QUARTER — RIGHT FRONT, RIGHT REAR, LEFT FRONT, LEFT REAR

## FIG. 2

Legend: ■ SOMATIC CELL COUNT □ SPECIFIC SURFACE AREA

Left axis: SOMATIC CELL COUNT/$10^4$ (0–900)
Right axis: SPECIFIC SURFACE AREA/$m^2g^{-1}$ (90–130)
X-axis: POSITION OF UDDER QUARTER — RIGHT FRONT, RIGHT REAR, LEFT FRONT, LEFT REAR

## FIG. 3

## FIG. 4

FIG. 5

SA-VB

STAPHYLOCOCCUS AUREUS STRAIN (0h, 3h, 6h, 9h)

FIG. 6

SA-VR

SA

FIG. 7

FERMENTATION TIME VS. SPECIFIC SURFACE AREA VALUE

## FIG. 8

## FIG. 9

101

400
MANAGEMENT TERMINAL

403
STORAGE SECTION

404
DISPLAY SECTION

402
CONTROL SECTION

405
NMR MEASURED VALUE COMPARISON SECTION

406
SOMATIC CELL COUNT COMPARISON SECTION

407
DETERMINATION SECTION

408
ABNORMALITY NOTIFICATION SECTION

401
COMMUNICATION SECTION

501
COMMUNICATION SECTION

502
CONTROL SECTION

503
MEASUREMENT SECTION

SOMATIC CELL MEASUREMENT DEVICE

500

301
COMMUNICATION SECTION

302
CONTROL SECTION

303
MEASUREMENT SECTION

304
CALCULATION SECTION

PULSED NMR DEVICE

300

200
MILKING MACHINE

FIG. 10

FIG. 11

EP 4 047 356 A1

# FIG. 12

EP 4 047 356 A1

EP 4 047 356 A1

## FIG. 13

### SA cultivation in Skim Milk at 37°C

**INTERNATIONAL SEARCH REPORT**

International application No.

PCT/JP2020/039008

A.  CLASSIFICATION OF SUBJECT MATTER
Int. Cl. C01N24/08(2006.01)i
FI: G01N24/08 510L, G01N24/08 510Q

According to International Patent Classification (IPC) or to both national classification and IPC

B.  FIELDS SEARCHED

Minimum documentation searched (classification system followed by classification symbols)
Int. Cl. G01N24/00-24/14, A01J5/00-5/16, A01J7/00-7/04, A61B8/00-8/15, G01N1/00-
1/44, G01N15/00-15/14, G01N21/00-21/83, G01N22/00-22/04, G01N23/00-23/2276,
G01N27/00-27/92, G01N29/00-29/52, G01N33/48-33/98, G01N35/00-35/10, G01N37/00

Documentation searched other than minimum documentation to the extent that such documents are included in the fields searched
Published examined utility model applications of Japan      1922-1996
Published unexamined utility model applications of Japan    1971-2020
Registered utility model specifications of Japan            1996-2020
Published registered utility model applications of Japan    1994-2020

Electronic data base consulted during the international search (name of data base and, where practicable, search terms used)
JSTPlus/JMEDPlus/JST7580 (JDreamIII), KAKEN, Science Direct, PubMed

C.  DOCUMENTS CONSIDERED TO BE RELEVANT

| Category* | Citation of document, with indication, where appropriate, of the relevant passages | Relevant to claim No. |
|---|---|---|
| X<br>Y | Nuclear magnetic resonance metabonomics reveals strong association between milk metabolites and somatic cell count in bovine milk, Journal of Dairy Science, January 2013, vo. 96, issue 1, pp. 290-299, doi: 10.3168/jds.2012-5819, "ABSTRACT", "MATERIALS AND METHODS" | 1, 3-4<br>2, 5-7 |
| Y | Rapid Surface Area Determination via NMR Spin-Lattice Relaxation Measurements, Powder Technology, November 1987, vol. 53, issue 1, pp. 39-47, doi: 10.1016/0032-5910(87)80123-7, in particular, "EXPERIMENTAL" | 2 |
| Y | JP 2012-021986 A (BRUKER BIOSPIN CORP.) 02 February 2012, fig. 1-3 | 5-7 |

☒  Further documents are listed in the continuation of Box C.        ☒  See patent family annex.

| | |
|---|---|
| *    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |

| Date of the actual completion of the international search<br>04.11.2020 | Date of mailing of the international search report<br>24.11.2020 |
|---|---|
| Name and mailing address of the ISA/<br>     Japan Patent Office<br>     3-4-3, Kasumigaseki, Chiyoda-ku,<br>     Tokyo 100-8915, Japan | Authorized officer<br><br>Telephone No. |

Form PCT/ISA/210 (second sheet) (January 2015)

| | INTERNATIONAL SEARCH REPORT | International application No. |
|---|---|---|
| | | PCT/JP2020/039008 |

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2014-521930 A (LIPOSCIENCE INC.) 28 August 2014, fig. 4-6 | 5-7 |
| Y | JP 2016-197104 A (BRUKER BIOSPIN GMBH) 24 November 2016, fig. 1-3 | 5-7 |
| A | The Effect of Lipopolysaccharide Induced Experimental Bovine Mastitis on Clinical Parameters, Inflammatory Markers, the Metabolome: A Kinetic Approach, Frontiers in Immunology, 25 June 2018, Volume 9, Article 1487, doi: 10.3389/fimmu.2018.01487, "MATERIALS, METHODS" | 1-7 |
| A | JP 2002-148957 A (FUJI XEROX CO., LTD.) 22 May 2002 | 1-7 |
| A | US 6288539 B1 (PHENO IMAGING, INC.) 11 September 2001 | 1-7 |

Form PCT/ISA/210 (continuation of second sheet) (January 2015)

**INTERNATIONAL SEARCH REPORT**
Information on patent family members

| International application No. |
|---|
| PCT/JP2020/039008 |

| Patent Documents referred to in the Report | Publication Date | Patent Family | Publication Date |
|---|---|---|---|
| JP 2012-021986 A | 02.02.2012 | US 2012/0092013 A1<br>fig. 1-3<br>US 2015/0102812 A1<br>fig. 1-3<br>EP 2407796 A1<br>fig. 1-3 | |
| JP 2014-521930 A | 28.08.2014 | US 2013/0002250 A1<br>fig. 4-6<br>US 2017/0293007 A1<br>fig. 4-6<br>WO 2013/003454 A2<br>fig. 4-6<br>EP 2726857 A2<br>fig. 4-6<br>AU 2012275478 A<br>CA 2840207 A1<br>CN 103930774 A<br>SG 10201608139V A | |
| JP 2016-197104 A | 24.11.2016 | US 2016/0290941 A1<br>fig. 1-3<br>EP 3076197 A1<br>fig. 1-3<br>DE 102015206030 B | |
| JP 2002-148957 A | 22.05.2002 | (Family: none) | |
| US 6288539 B1 | 11.09.2001 | US 6084407 A<br>WO 2001/081952 A1<br>WO 2000/016129 A1<br>AU 5379001 A<br>AU 5912099 A | |

Form PCT/ISA/210 (patent family annex) (January 2015)

**EP 4 047 356 A1**

**REFERENCES CITED IN THE DESCRIPTION**

**Patent documents cited in the description**

- JP 2002503097 W
- JP 2010230363 A
- JP 2010256231 A
- JP 2011033599 A